(19) Europäisches Patentamt
European Patent Office
Office européen des brevets

(11) Veröffentlichungsnummer: **0 054 166**
**B1**

(12) # EUROPÄISCHE PATENTSCHRIFT

(45) Veröffentlichungstag der Patentschrift: **09.05.84**

(21) Anmeldenummer: **81109212.1**

(22) Anmeldetag: **29.10.81**

(51) Int. Cl.³: **C 07 C 69/716,** C 07 C 67/313, C 07 C 121/34, C 07 C 120/00, C 07 C 67/54

(54) **Verfahren zur Herstellung von 5-Oxohexansäurealkylester und 5-Oxohexannitril.**

(30) Priorität: **17.12.80 DE 3047482**

(43) Veröffentlichungstag der Anmeldung: **23.06.82 Patentblatt 82/25**

(45) Bekanntmachung des Hinweises auf die Patenterteilung: **09.05.84 Patentblatt 84/19**

(84) Benannte Vertragsstaaten: **DE NL**

(56) Entgegenhaltungen:
**DE - A - 2 325 160**
**DE - B - 2 329 923**

(73) Patentinhaber: **HOECHST AKTIENGESELLSCHAFT, Postfach 80 03 20, D-6230 Frankfurt am Main 80 (DE)**

(72) Erfinder: **Müller, Werner Heinrich, Dr., Taunusblick 5, D-6239 Eppstein/Taunus (DE)**
Erfinder: **Popp, Knut, Hasenpfad 11, D-6232 Bad Soden am Taunus (DE)**
Erfinder: **Laugwitz, Bernd, Am Schieferberg 10d, D-6233 Kelkheim (Taunus) (DE)**

EP 0 054 166 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Es ist bekannt, dass man 5-Oxohexansäurealkylester und 5-Oxohexannitril durch die aminkatalysierte Addition von Aceton an Acrylsäurealkylester bzw. Acrylnitril herstellen kann (DE-OS 2 855 195).

Die 5-Oxohexansäurederivate sind wichtige Ausgangsstoffe für Cyclohexandion-(1,3), Resorcin und m-Aminophenole.

Ein wesentliches Problem des bekannten Verfahrens beruht in der schwierigen und mit hohen Verlusten verbundenen Reingewinnung der gewünschten Verbindungen aus den Reaktionsprodukten. Die Hauptreaktion und die wichtigsten Nebenreaktionen sind am Beispiel der Umsetzung von Aceton mit Methylacrylat in Gegenwart von Isopropylamin als Katalysator in Tabelle 1 zusammengestellt. Die aus der Umsetzung mit Acrylnitril resultierenden Substanzen lassen sich aus den Formelbildern durch Ersatz der Methylestergruppe durch die Nitrilfunktion ableiten.

Da das Aceton (AC) gegenüber dem Methylacrylat (MAC) bzw. Acrylnitril (AN) im grossen Überschuss verwendet wird und andererseits der Umsatz des Acrylats bzw. Acrylnitrils nach Gleichung (1) nicht vollständig ist, enthält das Reaktionsprodukt neben dem gewünschten 5-Oxohexansäuremethylester (OHM) bzw. 5-Oxohexansäurenitril (OHN) hauptsächlich unumgesetztes Aceton und Acrylat bzw. Acrylnitril. Ausserdem entstehen nach Gleichung (2) durch Zweifachaddition von Acrylverbindungen an Aceton Acetylheptandisäuredimethylester (AHDM) bzw. Acetyl-heptan-dinitril (AHDN). Durch Addition des Aminkatalysators an die Acrylverbindungen werden nach (3) β-Aminopropionsäuremethylester (IPM) bzw. -propionitril (IPN) gebildet. Durch Zweifachaddition von Acrylverbindung an den Aminkatalysator bilden sich nach (4) Aminodipropansäurederivate. Beim Einsatz von Methylacrylat wird durch teilweise Hydrolyse infolge der Einwirkung des nach Gleichungen (6) und (8) gebildeten Wassers Acrylsäure (ACS) und Methanol (MeOH) freigesetzt. Methanol addiert sich nach Gleichung (5) zu β-Methoxypropionsäure. Durch Acetonkondensation nach (6) entsteht Mesityloxid (MO) und Wasser. Durch Addition von Acrylverbindungen an Mesityloxid bilden sich nach (7) 5-Oxo-7-methyl-octan-6-säurederivate (OOM bzw. OON). Ausserdem ist noch die Kondensation von Aceton mit dem Aminkatalysator nach (8) unter Bildung der Schiffschen Base (IIPA) von Bedeutung.

Durch Hydrolyse des β-Aminopropionsäuremethylesters (IPM) nach Gleichung (9) entsteht die freie Aminosäure (IPS), welche als inneres Salz vorliegt. Da diese stark polare Substanz in organischen Medien unlöslich ist, kommt es zur Ausscheidung von Kristallen, wodurch Leitungen und Destillationskolonnen im Laufe der Zeit verstopfen. Ausserdem bildet z.B. die Isopropylaminopropionsäure (IPS) mit Hydrochinon eine schwerlösliche Additionsverbindung, was zur Ausfällung des Polymerisationsinhibitors führt.

Es braucht nicht eigens betont zu werden, dass eine Polymerisation der Acrylverbindung äusserst unerwünscht ist.

Tabelle 1

Bei der Umsetzung von Acrylsäuremethylester mit Aceton in Gegenwart von Isopropylamin ablaufende Reaktionen:

(1)

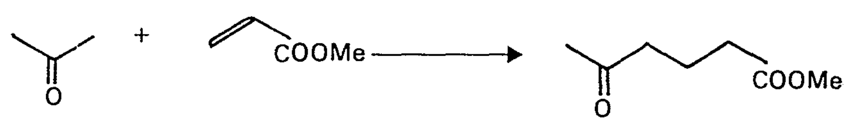

AC MAC OHM

(2)

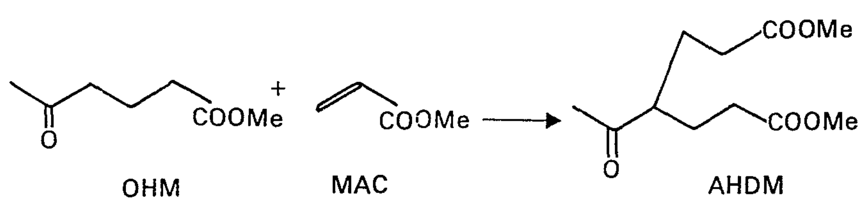

OHM MAC AHDM

(3)

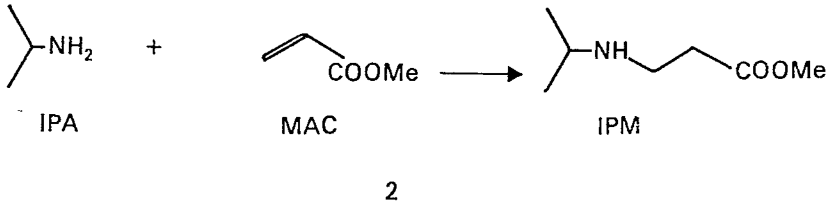

IPA MAC IPM

(4) 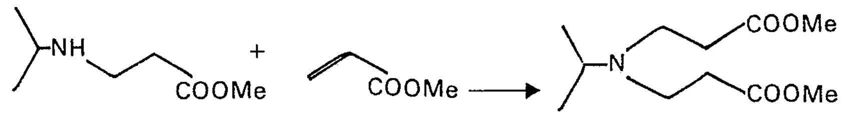

IPM MAC

(5) 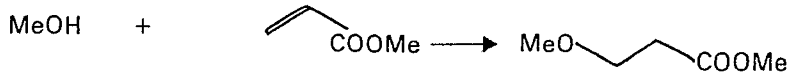

MAC

(6) 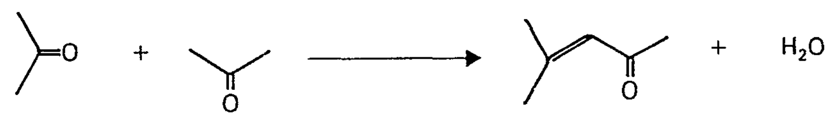

AC AC MO

(7) 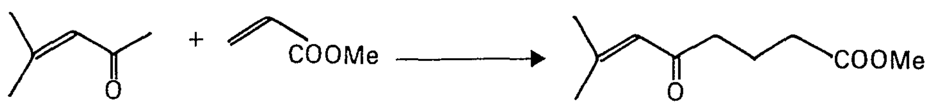

MO MAC OOM

(8) 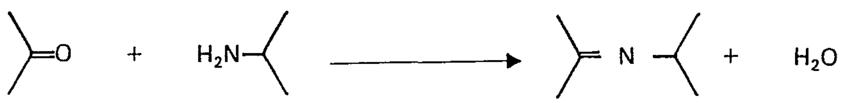

AC IPA IIPA

(9) 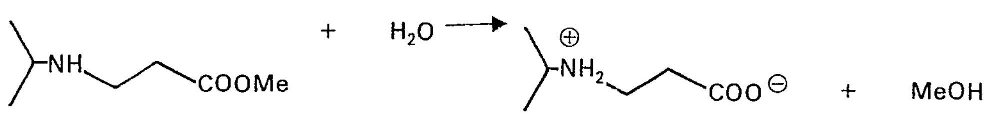

IPM IPA

Da bei einer Wasserkonzentration unter etwa 1% viele Nebenreaktionen, hauptsächlich die Acetylheptansäurederivatbildung (AHDM) nach (2), stark zunehmen, über 3% die Reaktionsgeschwindigkeit stark abnimmt, ist es notwendig, eine Wasserkonzentration zwischen diesen Werten einzustellen.

Da die Nebenreaktionen hauptsächlich bei niedriger Acetonkonzentration stattfinden, d.h. wenn das während der Reaktion in grossem Überschuss vorhandene Aceton bei der Aufarbeitung entfernt wird, müssen besondere Vorkehrungen getroffen werden, um die 5-Oxohexansäurederivate vor Folgereaktionen zu bewahren,

Die Isolierung von 5-Oxohexansäuremethylester aus den Reaktionsprodukten, die durch Addition von Aceton an Methylacrylat in Gegenwart von primären Aminkatalysatoren entstehen, wird in der US-Patentschrift 2 850 519 und der Europäischen Offenlegungsschrift 3 624 behandelt.

In der erstgenannten Literaturstelle wird beschrieben, dass es sich auf die Ausbeute des 5-Oxohexansäuremethylesters nachteilig auswirkt, wenn Katalysator und unumgesetzte Ausgangsmaterialien bei Temperaturen über 195°C abdestilliert werden, was jedoch notwendig ist, wenn bei atmosphärischem Druck gearbeitet wird, da dann der 5-Oxohexansäuremethylester erst bei etwa 212°C siedet und man daher im Sumpf mindestens diese Temperatur einstellen muss, um die Leichtsieder quantitativ abzutrennen.

Destilliert man dagegen im Vakuum um die Sumpftemperatur unter 195°C zu halten, so setzt sich nach Angaben dieser Literaturstelle bei Recyclierung des Destillats in den Reaktor am Rührer polymeres Material ab, was eine kontinuierliche Reaktionsführung verhindert. Gemäss der zitierten Europäischen Offenlegungsschrift werden diese Probleme dadurch überwunden, dass man bei einer Temperatur unter 195°C, mindestens Atmosphärendruck und in Gegenwart eines inerten Lösemittels mit einem Siedepunkt zwischen 80 und 195°C, die unumgesetzten Ausgangsmaterialien und den Katalysator abdestilliert. Als «inertes Lösungsmittel» wird dabei vorzugsweise Mesityloxid verwandt, da es als Nebenprodukt in dem erfindungsgemässen Verfahren durch Kondensation von zwei Molekülen Aceton unter Wasserabspaltung gebildet wird (Gleichung 6), und daher sowieso aus dem Rückstand, der nach Abtrennung der unumgesetzten Ausgangsmaterialien bleibt, abdestilliert werden muss.

Bei der Herstellung von 5-Oxohexansäuremethylester aus Aceton und Methylacrylat in Gegenwart von primären Aminen erhält man jedoch als eines der hochsiedenden Nebenprodukte nach Gleichung (7) 5-Oxo-7-methylocten-6-säuremethylester (OOM), d.h. das Additionsprodukt von Mesityloxid an Methylacrylat. Mesityloxid ist also unter den Reaktionsbedingungen keineswegs inert. Aus diesem Grunde sollte daher die Mesityloxidkonzentration besonders dann niedrig gehalten werden, wenn kein oder nur wenig Aceton für die Reaktion mit Methylacrylat zur Verfügung steht. Er erweist sich daher als besonders ungünstig, durch Rückführung von Mesityloxid gemäss der zitierten Europäischen Offenlegungsschrift den Destillationssumpf von Methylacrylat zu befreien, da dann in der Destillationskolonne Anreicherungszonen von Gemischen aus Mesityloxid und Methylacrylat auftreten.

Die in der Europäischen Offenlegungsschrift ebenfalls vorgeschlagene Verwendung eines systemfremden inerten Zwischensieders bedeutet ein weiteres Trennproblem, d.h. im kontinuierlichen Betrieb eine zusätzliche Destillationskolonne. Auch dieses Verfahren befriedigt somit nicht.

Als weiterer Nachteil des in der Europäischen Offenlegungsschrift 3624 beschriebenen Verfahrens erweist sich, dass bei der dort eingestellten Sumpftemperatur von 190°C das Reaktionswasser mit unumgesetztem Ausgangsmaterial abdestilliert wird.

Vor der Recyclierung des Ausgangsmaterials im kontinuierlichen Betrieb wird das Reaktionswasser, das sich hauptsächlich aus Aceton und Isopropylamin bei der Bildung von Isopropylidenisopropylamin (IIPA) bildet, durch Trocknung mit Molsieben aus dem Destillat entfernt. Dieses Verfahren ist umständlich und aufwendig.

Die aufgezeigten Probleme treten auch bei der Verwendung von höherem Acrylester bzw. von Acrylnitril als Ausgangsmaterialien auf.

Es war daher notwendig, Verfahrensbedingungen bei der Aufarbeitung des Reaktionsgemisches aufzufinden, unter welchen sowohl eine Ausbeuteminderung durch Weiterreaktion des gewünschten 5-Oxohexansäurealkylester bzw. -nitrils zu Hochsiedern als auch die verfahrenstechnisch problematische Hydrolyse der β-Aminopropionsäureester bzw. -nitrile zur unlöslichen freien Aminosäure durch eine möglichst frühzeitige Trennung und Abzweigung der reaktionsfähigen Komponenten zurückgedrängt werden. Diese Gesichtspunkte sind von besonderer Bedeutung bei kontinuierlichen Herstellverfahren, da in diesem Fall die Gefahr besteht, dass sich schädliche Komponenten wie Wasser, Mesityloxid oder freie Aminopropionsäure im Kreislauf der zurückgewonnenen und wieder eingesetzten Ausgangsstoffe anreichern und zu Störungen im Produktionsablauf führen.

Es bestand daher die Aufgabe, ein kontinuierliches Verfahren zur Herstellung von 5-Oxohexansäurealkylester bzw. -nitril zu entwickeln, bei dem die unumgesetzten Ausgangsmaterialien nach ihrer Abtrennung verlustlos recycliert werden können, der synthetisierte 5-Oxohexansäurealkylester bzw. das -nitril ohne Verlust infolge Zersetzung oder Weiterreaktion isoliert werden kann, sowie das Reaktionswasser und andere Nebenprodukte durch Ausschleusung auf einer bestimmten niedrigen Konzentration gehalten werden können. Ein weiteres Ziel der Erfindung was das Auffinden einer einfachen Abtrennungsmöglichkeit für die Additionsprodukte von primären Aminen an Acrylsäureester bzw. Acrylnitril, welche in ihrem Siedepunkt zwischen Mesityloxid und 5-Oxohexansäurederivat liegen. Diese Aminopropionsäurederivate können direkt oder nach Spaltung recycliert werden und verringern daher den Verbrauch von Isopropylamin und Acrylester bzw. Acrylnitril erheblich.

Es wurde nun ein Verfahren gefunden zur Herstellung von 5-Oxohexansäurealkylester bzw. 5-Oxohexannitril durch Umsetzung von Aceton mit einem Acrylester, dessen Alkoholkomponente eine Kettenlänge von 1 bis 6 C-Atomen aufweist, bzw. mit Acrylnitril bei erhöhter Temperatur in der Flüssigphase in Gegenwart eines primären Amins und/oder einer Schiffschen Base aus primären Amin und Aceton und/oder eines β-Aminopropionsäurederivates aus primärem Amin und Acrylester bzw. Acrylnitril sowie einer sauren Verbindung, bei einem Druck oberhalb des Dampfdrucks des Reaktionsgemisches und unterhalb 500 bar, welches dadurch gekennzeichnet ist, dass das Reaktionsgemisch nach der Umsetzung kontinuierlich in eine Destillationskolonne eingespeist wird, über Kopf die Leichtsieder abdestilliert werden, aus der sich bildenden Anreicherungszone des Mesityloxid-Wasser-Azeotrops dieses abgezogen und aus dem zurückbleibenden Gemisch der 5-Oxohexansäurealkylester bzw. das 5-Oxohexannitril destillativ in derselben oder einer weiteren Kolonne gewonnen wird.

Geeignete Ausgangsstoffe sind Acrylester von $C_1$- bis $C_6$-Alkoholen, vorzugsweise Methanol und Ethanol, sowie Acrylnitril. Besonders bevorzugt werden Methylacrylat und Acrylnitril.

Die Menge des zugesetzten Katalysators beträgt im allgemeinen zwischen 0,01 und 0,4 Mol pro Mol Acrylverbindung.

Als Katalysatoren geeignet sind z.B. Methylamin, Ethylamin, n-Propylamin, Isopropylamin, n-Butylamin, Isobutylamin, sek.-Butylamin, tert.-Butylamin, Amylamin, Isoamylamin, Cyclohexylamin, Cyclopentylamin, Benzylamin sowie deren Schiffsche Basen mit Aceton und die Additionsprodukte der genannten Amine an Alkylacrylat bzw. Acrylnitril (= β-Aminopropionsäurederivate).

In einer bevorzugten Ausführungsform der Erfindung wird der in einer Zone unterhalb der Anreicherungszone des Mesityloxid-Wasser-Azeotrops sich anreichernde β-Aminopropionsäurealkylester bzw. das entsprechende Nitril ebenfalls aus der erstgenannten Kolonne abgezogen.

Hierfür besonders geeignete Katalysatoren sind z.B. Methylamin, Ethylamin, n-Propylamin, Isopropylamin und Butylamine, deren Schiffsche Basen mit Aceton sowie die Additionsprodukte der genannten Amine an Alkylacrylat bzw. Acrylnitril, da deren Siedepunkte zwischen denen von Mesityloxid und 5-Oxohexansäurealkylester bzw. -nitril liegen.

Als saure Verbindung wird im allgemeinen eine organische Säure, beispielsweise Acrylsäure, Methacrylsäure, Crotonsäure, Essigsäure, Propionsäure, Hexansäure, 5-Oxohexansäure, Ethylhexansäure, Benzoesäure, 2-Nitrobenzoesäure, N-Dialkylaminpropionsäure oder ein Gemisch, das eine oder mehrere dieser Säuren enthält, beispielsweise der Destillationsrückstand aus der 5-Oxohexansäurealkylester-Reindestillation, verwendet. Die Konzentration der Säure beträgt im allgemeinen 0,01 bis 2 Gew.-%, vorzugsweise 0,1 bis 0,5 Gew.-%, bezogen auf das Reaktionsgemisch.

Das molare Verhältnis von Aceton zu Acrylester bzw. Acrylnitril beträgt im allgemeinen 2:1 bis 20:1, vorzugsweise 3:1 bis 8:1.

Die Umsetzung kann mit oder ohne Lösungs- oder Verdünnungsmittel ausgeführt werden. Besonders bewährt hat sich – falls Aceton mit Acrylsäuremethylester umgesetzt wird – die Anwesenheit von 1 bis 20 Gew.-%, vorzugsweise 5 bis 15 Gew.-% Methanol, bezogen auf das Reaktionsgemisch. Es hat sich nämlich gezeigt, dass bei einer Methanolkonzentration von etwa 5 Gew.-% im Reaktionsgemisch eine weitere Zunahme der Methanolkonzentration im kontinuierlichen Betrieb, d.h. unter Rückführung aller nicht umgesetzten Ausgangsmaterialien, unterbleibt. Methanol bildet ausserdem mit Methylacrylat (Kp. 80,5°C) ein Azeotrop vom Kp. 62°C und erleichtert deshalb die destillative Abtrennung des unumgesetzten Methylacrylats aus dem Reaktionsprodukt erheblich. Ein analoges Azeotrop bildet sich bei Einsatz von Ethylacrylat sowohl mit Methanol (Kp. 64,5°C) als auch mit Ethanol (Kp. 77,5°C).

Zweckmässig ist im allgemeinen der Zusatz von Polymerisationsinhibitoren wie Hydrochinon, Hydrochinonmonomethylether oder Phenothiazin, um eine radikalische Polymerisation der Acrylverbindung zu verhindern.

Die Reaktionstemperatur beträgt im allgemeinen 150 bis 235°C, vorzugsweise 200 bis 235°C; unter 150°C ist die Reaktionsgeschwindigkeit sehr gering, über 235°C, der kritischen Temperatur des Acetons, fällt die Reaktionsgeschwindigkeit ebenfalls ab. Der Druck wird durch Inertgasüberlagerung vorzugsweise auf einen Wert von 2 bis 300 bar, insbesondere 5 bis 200 bar über den der Reaktionstemperatur entsprechenden Dampfdruck eingestellt.

Das den Reaktionsraum verlassende Produktgemisch enthält noch einen beträchtlichen Anteil an unumgesetzten Ausgangsstoffen (Aceton, Acrylverbindungen) sowie leichtflüchtigen Nebenprodukten (Isopropylidenamin aus der Umsetzung von Aceton und primärem Amin, gegebenenfalls Alkanol wie z.B. Methanol), welche bei der nachfolgenden destillativen Aufarbeitung als sogenannte Leichtsieder anfallen. Sie werden zur erneuten Umsetzung direkt in den Reaktionsraum recycliert. Die Auftrennung des Reaktionsgemisches erfolgt in einer Destillationskolonne, z.B. einer Glockenboden- oder Füllkörperkolonne, wobei die Leichtsieder mit einem Siedepunkt unter 90°C bei Normaldruck über Kopf abdestilliert werden. Im Oberteil der Kolonne reichert sich ein azeotropes Gemisch aus Mesityloxid und Wasser an, welches seitlich abgezogen und aus dem Prozess entfernt wird. Das Mesityloxid kann auch in Aceton zurückgespalten werden und als Ausgangsstoff erneut eingesetzt werden.

In einer tiefer liegenden Zone der Destillationskolonne reichert sich 5-Oxohexansäurealkylester bzw. -nitril an und kann dort ebenfalls – vorteilhaft aus der Gasphase – seitlich abgezogen werden.

Bevorzugt wird diese Substanz jedoch zusammen mit den anfallenden Hochsiedern als Sumpfprodukt abgezogen und in einer nachgeschalteten Kolonne aufgetrennt, wobei über Kopf der reine 5-Oxoalkansäurealkylester bzw. das -nitril abdestilliert.

Die erstgenannte Destillationskolonne kann bei Normaldruck oder unter leichtem Vakuum bis herab zu etwa 20 mbar und einer Sumpftemperatur von 100 bis 200°C betrieben werden.

Die zweite Kolonne wird vorzugsweise im Vakuum bei einem Druck von 1 bis 20 mbar und einer Sumpftemperatur zwischen 100 und 200°C betrieben.

Zur Verbesserung der Ausbeute ist es günstig, aus der erstgenannten Destillationskolonne in einem unterhalb der Anreicherungszone des Mesityloxid-Wasser-Azeotrops gelegenen Bereich den sich dort anreichernden Alkylester bzw. das Nitril der β-Aminopropionsäure seitlich abzuziehen und entweder direkt oder nach Spaltung in Amin und Acrylverbindung in den Reaktionsraum zurückzuführen. Durch die schonende Behandlung lässt sich eine Zersetzung bzw. Weiterreaktion dieser empfindlichen Stoffe z.B. in Polyamide vermeiden und dadurch die Reaktionsausbeute erhöhen.

Eine weitere, bevorzugte Ausführungsform der Erfindung besteht darin, dass das Reaktionsgemisch nach der Umsetzung

a) von der Hauptmenge der bei Normaldruck unter 90°C siedenden Komponenten befreit wird, welche direkt in den Reaktionsraum zurückgeführt werden, und sodann

b) kontinuierlich in eine Destillationskolonne eingespeist wird, wo über Kopf der Rest der unter 90°C siedenden Komponenten abgenommen und in den Reaktionsraum zurückgeführt wird,
c) aus der Anreicherungszone des Mesityloxid-Wasser-Azeotrops dieses abgezogen und aus dem Prozess ausgeschleust wird,
d) aus der unterhalb der unter c) genannten Zone liegenden Anreicherungszone von β-Aminopropansäuremethylester bzw. β-Aminopropannitril diese Verbindung abgezogen und direkt oder nach Spaltung in Amin und Acrylverbindung in den Reaktor zurückgeführt wird,
e) aus dem Rückstand verbleibenden Substanzgemisch der 5-Oxohexansäuremethylester bzw. das 5-Oxohexannitril in derselben oder einer nachgeschalteten Vakuumkolonne destillativ gewonnen wird.

Die Abtrennung der Hauptmenge der Leichtsieder in Schritt a) kann beispielsweise nach Abkühlung des Reaktionsgemisches über eine bei Normaldruck und einer Sumpftemperatur von 60 bis 100°C betriebene Destillationskolonne über Kopf vorgenommen werden. Günstiger ist es jedoch, das noch heisse, unter Druck stehende Reaktionsgemisch in eine Kolonne oder einen Verdampfer zu entspannen, wobei die Hauptmenge der Leichtsieder abdestilliert und das Sumpfprodukt in die in den Schritten b), c) und d) eingesetzte Destillationskolonne eingespeist wird, wo der Rest der Leichtsieder unter Normaldruck oder leichtem Vakuum, vorzugsweise im Bereich von 20 bis 130 mbar und einer Sumpftemperatur von 100 bis 200°C über Kopf abgetrennt wird.

Besonders bevorzugt wird eine Verfahrensweise, welche darin besteht, dass das Reaktionsgemisch nach der Umsetzung

a) kontinuierlich in einem Verdampfer zwischen 60 und 100°C vom Grossteil der Leichtsieder befreit wird, welche direkt recycliert werden,
b) sodann in eine bei 20 bis 130 mbar betriebenen Vakuumkolonne eingespeist wird, wo über Kopf der Rest der Leichtsieder abgenommen und recycliert wird,
c) in einer Zone unmittelbar über dem Kolonnenzulauf Aminopropansäuremethylester bzw. Aminopropansäurenitril kontinuierlich abgenommen und direkt oder nach Zersetzung in Amin und Acrylverbindung recycliert wird,
d) oberhalb der Anreicherungszone vom Aminopropansäureester oder -nitril ein Azeotrop aus Mesityloxid und Wasser abgenommen und aus dem Prozess ausgeschleust wird,
e) das im Sumpf dieser Kolonne anfallende Produkt kontinuierlich einer weiteren Vakuumkolonne zugeführt wird, wo über Kopf reiner 5-Oxohexansäuremethylester bzw. 5-Oxohexannitril anfällt und im Sumpf hauptsächlich Acetylheptandisäuredimethylester bzw. -dinitril sowie 5-Oxo-7-methylocten-6-säure-methylester bzw. -nitril anfallen.

Wie oben ausgeführt, wird das Mesityloxid-Wasser-Azeotrop entweder in der ersten auf das Reaktionsgefäss folgenden Kolonne abgetrennt oder – falls der Hauptteil der Leichtsieder vorher entfernt wird – in der ersten auf diese Entfernung folgenden Kolonne. Die Kolonne, aus welcher das Mesityloxid-Wasser-Azeotrop abgezogen wird, soll im folgenden als «MO-Kolonne» bezeichnet werden.

Die vorliegende Erfindung eröffnet die Möglichkeit, die beiden unerwünschten Nebenprodukte Wasser und Mesityloxid kontinuierlich aus dem Prozess zu entfernen. Es war nicht vorhersehbar, dass sich diese beiden Stoffe bei der destillativen Aufarbeitung des Vielkomponentengemisches der Reaktion in einer bestimmten Zone der MO-Kolonne anreichern würden. Zwar ist bekannt, dass diese beiden Stoffe ein bei 91,8°C siedendes Azeotrop bilden. Jedoch war nicht zu erwarten, dass in Gegenwart einer Vielzahl anderer, zum Teil mit Wasser mischbarer Substanzen wie Aceton, Aminverbindungen, Alkanol, Acrylsäurederivat usw. eine Auftrennung in dieses Azeotrop erfolgen würde. Es hat sich als vorteilhaft erwiesen, dieses Azeotrop aus der Kolonne abzuziehen und entweder aus dem Prozess auszuschleusen oder das Mesityloxid in Aceton zurückzuspalten und dieses erneut als Ausgangsstoff einzusetzen.

Beim Einsatz höhersiedender Acrylester, wie z.B. Butylacrylat, kann die Rückgewinnung des unumgesetzten Teils ebenfalls durch Abzweigung aus der Anreicherungszone des Acrylats in der MO-Kolonne erfolgen.

In einer weiter unten gelegenen Zone der MO-Kolonne reichert sich 5-Oxohexansäurealkylester bzw. 5-Oxo-hexannitril an und kann dort gleichfalls abgezweigt werden. Bevorzugt wird der gesamte aus der MO-Kolonne anfallende Sumpf einer weiteren Kolonne zugeführt, welche vorzugsweise im Vakuum, insbesondere bei einem Druck zwischen 1 und 20 mbar, über Kopf reinen 5-Oxohexansäurealkylester bzw. 5-Oxohexannitril liefert, während aus einer Zone knapp oberhalb des Sumpfes 5-Oxo-7-methyl-octen-6-säurealkylester bzw. 5-Oxo-7-methyl-octen-6-säurenitril abgenommen werden kann und im Sumpf hauptsächlich Acetylheptansäuredialkylester bzw. -dinitril anfallen.

Der Vorteil des erfindungsgemässen Verfahrens besteht darin, dass die reaktiven Komponenten des aufzuarbeitenden Reaktionsgemisches so schnell voneinander getrennt werden, dass sie nicht mehr in nennenswertem Masse miteinander zu den unerwünschten Nebenprodukten reagieren können.

Beispielsweise werden Wasser und Aminopropansäureester in unterschiedlichen Zonen der MO-Kolonne angereichert und dort entnommen, so dass sie sich nicht mehr zur schwerlöslichen freien Aminosäure umsetzen können. Eine Abscheidung dieses Feststoffes in der Destillationskolonne würde diese in kurzer Zeit ausser Funktion setzen.

Die frühzeitige Trennung des Mesityloxids von den leichtsiedenden Acrylverbindungen verhindert die Bildung der unerwünschten Hochsieder (OOM bzw. OON).

Bedingt durch die Unterdrückung der Nebenreaktionen sind die nach den erfindungsgemässen Aufarbeitungsverfahren erzielten Ausbeuten erheblich höher als nach dem Stand der Technik.

Ausserordentlich vorteilhaft ist, dass nach der erfindungsgemässen, kontinuierlichen Abtrennung des Mesityloxid-Wasser-Azeotrops der Wassergehalt der Reaktionsmischung sich im optimalen Bereich zwischen 1 bis 3 Gew.-% bewegt, der für eine hohe Raum-Zeit-Ausbeute wesentlich ist. Die aufwendige und umständliche Entfernung des überschüssigen Wassers mittels Molekularsieb, die in der Europäischen Offenlegungsschrift 3624 beschrieben ist, wird damit überflüssig.

Die in der US-PS 2 850 519 geschilderte Abscheidung von polymerem Material bei der Recyclierung der im Vakuum abgetrennten Destillate in den Reaktor tritt beim erfindungsgemässen Verfahren nicht auf.

Die folgenden Beispiele dienen der Erläuterung der Erfindung.

Beispiele

In den nachfolgenden Beispielen werden die Substanzbezeichnungen wie folgt abgekürzt:

| | |
|---|---|
| Aceton | AC |
| Acrylsäuremethylester | MAC |
| Acrylnitril | AN |
| Acrylsäure | ACS |
| 4-Acetyl-heptandisäuredimethylester | AHDM |
| 4-Acetyl-heptandisäurenitril | AHDN |
| Isopropylamin | IPA |
| Isopropyliden-isopropylamin | IIPA |
| β-Isopropylamino-propansäure-methylester | IPM |
| β-Isopropylamino-propionitril | IPN |
| β-Isopropylamino-propionsäure | IPS |
| Mesityloxid | MO |
| Methanol | MeOH |
| 5-Oxohexansäuremethylester | OHM |
| 5-Oxohexannitril | OHN |
| 5-Oxo-7-methylocten-6-säure-methylester | OOM |
| 5-Oxo-7-methylocten-6-nitril | OON |

Die Struktur der aufgelisteten Substanzen ist aus Tabelle 1 ersichtlich bzw. abzuleiten.

Vergleichsbeispiel 1

In diesem Vergleichsbeispiel soll dargelegt werden, welche Ausbeuteverluste an OHM nach dem bisher üblichen Aufarbeitungsverfahren der diskontinuierlichen Destillation des Reaktionsansatzes [aus der Umsetzung von MAC mit AC in Gegenwart von IPA als Katalysator] hingenommen werden müssen.

Destilliert man das Reaktionsprodukt der folgenden typischen Zusammensetzung (gaschromatographisch bestimmt):

| Verbindung | AC | MAC | MeOH | MO | IPM | OHM | AHDM | $H_2O$ |
|---|---|---|---|---|---|---|---|---|
| Gew.-% | 57 | 3 | 11 | 2 | 3 | 18 | 2 | 2 |
| Sdp. °C/mbar | 56/1000 | 81/1000 | 65/1000 | 65/1000 | 172/1000 | 92/13 | 170/13 | 100/1000 |

diskontinuierlich, indem man zuerst bei Normaldruck bis zu einer Sumpftemperatur von 140°C die Leichtsieder AC, MAC und MeOH abdestilliert und anschliessend bei 10 bis 40 mbar die Mittelsieder MO, IPM und $H_2O$, so kann man bei 10 mbar schliesslich nur noch etwa 70 bis 80% des ursprünglich synthetisierten OHM destillativ gewinnen. Der Rest geht unter Bildung von Hochsiedern verloren.

Ausserdem können nur etwa 20 bis 30% des IPM zusammen mit MO und $H_2O$ abgetrennt werden. Versucht man, dieses Gemisch in einer weiteren Destillation zu trennen, so findet im Kolonnensumpf Hydrolyse von IPM unter Bildung von freier IPS statt. Das bedeutet nicht nur Verlust von IPM, das ja bekanntlich als Katalysator wieder zur Reaktionsmischung zurückgeschleust werden könnte, sondern wegen der Schwerlöslichkeit von IPS ein grosses verfahrenstechnisches Problem.

Vergleichsbeispiel 2

In diesem Vergleichsbeispiel wird belegt, dass auch eine kontinuierliche Destillation des Reaktionsgemisches nach dem Stand der Technik zu Verlusten an Ausgangsstoffen, Katalysator und am Endprodukt führt. Zur Veranschaulichung der verwendeten Apparatur dient Figur 1.

Aus einem Mischgefäss (1), welches über die Rohrleitungen (2), (3) und (4) mit den Ausgangsmaterialien sowie über (5) mit zurückgewonnenen Ausgangsmaterialien versorgt wird, werden pro Stunde 9,4 kg eines Gemisches der Zusammensetzung

| | Ausgangs-mischung | AC | MAC | MeOH | $H_2O$ | MO | IPM | IIPA | ACS |
|---|---|---|---|---|---|---|---|---|---|
| Gew.-% | | 64,0 | 19,6 | 10,4 | 1,7 | 0,6 | 1,8 | 1,7 | 0,2 |
| g/h | 9400 | 6016 | 1840 | 978 | 160 | 57 | 170 | 160 | 19 |

über Leitung (6) durch ein als Reaktor dienendes Strömungsrohr (7) von 12 l Inhalt, an dessen Ende sich eine Stand- und Druckhaltung befinden und das mittels einer Ölheizung auf 225°C erhitzt wird, gepumpt. Der Reaktor wird mittels Stickstoffüberlagerung auf 50 bar eingestellt.

Das am Reaktorausgang stündlich anfallende Produkt hat laut gaschromatographischer Analyse folgende Zusammensetzung:

| | Reaktionsprodukt | AC | MAC | MeOH | $H_2O$ | MO | IPM | IIPA | OHM | OOM | AHDM |
|---|---|---|---|---|---|---|---|---|---|---|---|
| Gew.-% | | 56,7 | 9,0 | 10,3 | 1,9 | 1,1 | 1,8 | 1,4 | 14,7 | 0,5 | 1,7 |
| g/h | 9400 | 5330 | 846 | 968 | 180 | 103 | 169 | 132 | 1382 | 47 | 160 |

Das Reaktionsprodukt wird über die Leitung (8) in die Mitte einer 2 m langen Destillationskolonne (9) mit einem Durchmesser von 10 cm eingespeist und dort bei atmosphärischem Druck, einer Sumpftemperatur von 140°C und einem Rücklaufverhältnis von 0,2 aufgetrennt. Das Destillat dieser Kolonne (Destillat 1) hat folgende Zusammensetzung:

| | Destillat 1 | AC | MAC | MeOH | $H_2O$ | IIPA |
|---|---|---|---|---|---|---|
| Gew.-% | | 72,2 | 10,1 | 13,9 | 2,1 | 1,7 |
| g/h | 7240 | 5230 | 734 | 1008 | 150 | 123 |

Das Destillat wird über die Leitung (5) in das Mischgefäss (1) recycliert. Das Sumpfprodukt = Sumpf 1 hat folgende Zusammensetzung:

| | Sumpf 1 | AC | MAC | $H_2O$ | MO | IPM | OHM | OOM | AHDM | unbek. Hochsieder |
|---|---|---|---|---|---|---|---|---|---|---|
| Gew.-% | | 3,1 | 2,1 | 1,6 | 4,6 | 3,4 | 58,3 | 2,1 | 8,3 | 16,3 |
| g/h | 2160 | 68 | 46 | 35 | 100 | 73 | 1260 | 45 | 180 | 365 |

Man erkennt, dass während dieser Destillation stündlich 66 g MAC (= 7,8%), 96 g IPM (= 57%) und 122 g OHM (= 8,9%) der im Reaktionsgemisch ursprünglich vorhandenen MAC-, IPM- und OHM-Mengen unter Bildung hochsiedender, durch die Gaschromatographie nicht erfassbarer Substanzen verlorengehen.

Der Sumpf 1 wird über Leitung (10) kontinuierlich in eine zweite Kolonne (11) bestehend aus 20 Glockenböden mit einem Durchmesser von 5 cm eingespeist. Der Zulauf befindet sich zwischen dem 10. und 11. Boden. Die Kolonne wird bei 20 mbar Kopfdruck und einem Rücklauf von $V_R = 0,5$ bei einer Sumpftemperatur von 115°C und einer Kopftemperatur zwischen 35 und 45°C betrieben. Dabei fallen stündlich über Leitung (12) ein Destillat = Destillat 2 und ein Sumpfprodukt = Sumpf 2 der folgenden Zusammensetzung an:

| | Destillat 2 | MAC | MO | IPM | OHM | $H_2O$ |
|---|---|---|---|---|---|---|
| Gew.-% | | 10 | 49 | 20 | 10 | 11 |
| g/h | 200 | 20 | 98 | 40 | 20 | 22 |

| | Sumpf 2 | IPM | OHM | OOM | AHDM | unbek. Hochsieder |
|---|---|---|---|---|---|---|
| Gew.-% | | 0,5 | 65,6 | 2,7 | 10,2 | 21 |
| g/h | 1860 | 10 | 1220 | 50 | 190 | 390 |

Ausserdem fallen stündlich etwa 90 g Substanz – im wesentlichen Aceton, Wasser und MAC – in den mit Trockeneis/Methanol gekühlten Kältefallen vor der Vakuumpumpe an.

Der Sumpf 2 wird kontinuierlich über Leitung (13) in eine 2,70 m lange 3. Kolonne (14) mit einem Durchmesser von 5 cm eingespeist. Die Kolonne ist mit einem Metallgeflecht (Goodloe-Packung) gefüllt und wird bei 13 mbar betrieben. Die Sumpftemperatur beträgt 170°C, die Kopftemperatur 98°C. Der Zulauf (13) liegt 1,70 m über dem Sumpf. Das Destillat (1200 g/h) = Destillat 3, das über Leitung (15) anfällt, besteht zu 98% aus OHM. Das über Leitung (16) abfliessende Sumpfprodukt = Sumpf 3 enthält neben AHDM hauptsächlich unbekannte Hochsieder.

Das Destillat 2 mit der oben angegebenen Zusammensetzung lässt sich destillativ nicht unzersetzt trennen. Es findet sofort Hydrolyse des IPM zu Isopropylaminopropansäure statt, welche im organischen Medium schwer löslich ist und zur Verstopfung der Leitungen und der Destillationskolonne führt.

Versucht man, es undestilliert in den Reaktionsraum zurückzuführen, so reichert sich im Reaktionsgemisch MO an, was zu einer vermehrten Bildung von Hochsiedern (OOM) führt. Durch das zusätzlich zugeführte Wasser steigert sich der Wassergehalt schnell über die Grenze von 3 bis 5% hinaus, wodurch die Reaktionsgeschwindigkeit stark abnimmt.

Beispiel 1

Herstellung von 5-Oxohesansäuremethylester

Beispiel 1 zeigt, wie durch das erfindungsgemässe Verfahren die im Vergleichsbeispiel 2 aufgezeigten Probleme gelöst werden. Zur Veranschaulichung dient Figur 2.

In das Mischgefäss (1) werden pro Stunde über Leitung (2) 1,10 kg MAC, 8 g ACS und 4 g Hydrochinon sowie über Leitung (3) 0,74 kg AC und über Leitung (4) 62 g Isopropylamin frisch zugeführt. Dazu kommen folgende Recyclierungsströme:

über Leitung (5) 4,7 kg AC, 0,8 g MAC, 0,9 kg MeOH, 45 g MO, 35 g IPM und 140 g $H_2O$;

über Leitung (17) 0,6 kg hauptsächlich AC und MAC;

über Leitung (18) 0,23 kg, bestehend hauptsächlich aus IPM neben geringen Mengen MO und OHM.

Nach intensivem Mischen im Mischgefäss (1) wird das gebildete Ausgangsgemisch (9,4 kg/h bestehend aus 64% AC, 20% MAC, 10,5% MeOH, 0,6% MO, 1,8% IPM, 1,7% $H_2O$ und 1,8% IIPA) über Leitung (6) in ein als Reaktor dienendes Strömungsrohr (7) von 12 l Inhalt gepumpt. Die Reaktortemperatur beträgt 230°C, der Druck 55 bar. Das am Reaktorende stündlich anfallende Gemisch (57% AC, 9,5% MAC, 10,3% MeOH, 1,1% MO, 1,8% IPM, 15,8% OHM, 1,7% AHDM, 1,9% $H_2O$, 1,4% IIPA) wird über Leitung (19) direkt in den Verdampfer (20) entspannt, der bei Normaldruck und etwa 85°C Sumpftemperatur betrieben wird. Dabei entsteht ein Destillat, das über Leitung (5) zum Mischgefäss (1) recycliert wird und die oben im Zusammenhang mit Leitung (5) genannte Zusammensetzung hat. Im Sumpf des Verdampfers (20) fallen stündlich 2,8 kg eines Gemisches der folgenden Zusammensetzung an: AC 20%; MAC 6%; MeOH 2%; MO 2%; IPM 5,5%; OHM 53%; OOM 0,3%, AHDM 6%; $H_2O$ 1%.

Dieses Gemisch wird über Leitung (21) kontinuierlich in die Kolonne (22) eingespeist. Diese besteht im Abtriebsteil aus einer 1,5 m Füllkörperkolonne mit Goodloe-Packung. Der Verstärkungsteil besteht aus 20 Glockenböden mit 5 cm Durchmesser. Der Druck am Kolonnenkopf beträgt 50 mbar; die Kopftemperatur beträgt −5°C, die Sumpftemperatur wird auf 144°C eingestellt. Das Rücklaufverhältnis beträgt $V_R = 3$. Am Kolonnenkopf, der mit Sole gekühlt wird, fallen stündlich etwa 0,6 kg eines Gemisches an, das hauptsächlich aus AC, MAC und etwas MeOH besteht und wie oben beschrieben über Leitung (17) zum Mischgefäss (1) zurückgeführt wird. Auf dem 2. Boden über dem Zulauf werden stündlich etwa 0,23 kg eines Gemisches abgenommen, das zu 75% aus IPM besteht, neben etwas OHM und MO. Da IPM als Katalysator wirkt, wird dieses Gemisch direkt über Leitung (18) ins Mischgefäss recycliert. Durch Abnahme dieses Gemisches unmittelbar über dem Kolonnenzulauf wird für eine kurze Verweilzeit des zersetzlichen IPM gesorgt. Ausserdem gelingt so die Abtrennung von Wasser-MO-Azeotrop, das 8 Böden oberhalb von Leitung (18) (IPM-Seitenabnahme) als 2-phasiges Gemisch über Leitung (23) ausgeschleust wird. Es fallen dabei stündlich etwa 120 g an, die neben MO und Wasser noch geringe Mengen β-Methoxypropionsäuremethylester enthalten. Durch Ausschleusen dieses Gemisches gelingt es, den Wasseranteil im Ausgangsgemisch im Mischgefäss (1) zwischen 1 bis 3 Gew.-% zu regeln, was für eine optimale Ausbeute ohne Abfall der Raumzeitleistung notwendig ist. Aus diesem MO-Wasser-Azeotrop lässt sich entweder reines MO gewinnen, oder es kann nach Spaltung in AC wieder zum Mischgefäss (1) zurückgeführt werden.

Im Sumpf von Kolonne (22) fallen stündlich 1,85 kg eines Gemisches an, das abgesehen von 0,2% IPM völlig frei von Leichtsiedern ist. Neben 82,5% OHM sind darin 10,5% AHDM und 1,2% OOM enthalten.

Dieses Gemisch wird über Leitung (24) kontinuierlich in die 2,70 m lange Kolonne (14) mit 5 cm Durchmesser eingespeist. Diese Kolonne ist mit einem Metallgeflecht (Goodloe-Packung) gefüllt und wird bei 13 mbar Druck am Kolonnenkopf betrieben. Die Sumpftemperatur beträgt 170 bis 180°C. Der Zulauf (24) zur Kolonne (14) befindet sich 1,70 m über dem Sumpf. Am Kopf werden stündlich 1,5 kg 99%iges OHM bei einem Rücklaufverhältnis von $V_R = 0{,}1$ Leitung (15) abgenommen.

Im Sumpf der Kolonne (14) fallen stündlich 0,28 kg eines Hochsiedergemisches an, das u.a. 140 g AHDM und 20 g OOM enthält und über Leitung (16) entnommen wird.

In einem Nebenversuch wurden aus einer Zone direkt oberhalb des Sumpfes der Destillationskolonne ca. 20 g OOM pro Stunde abgezogen.

Aus den stündlich dem System zugeführten Mengen und dem stündlich anfallenden OHM errechnen sich folgende Selektivitäten bezüglich AC bzw. MAC:

| Ausgangsmaterialien: | eingesetzt kg/h | Mol/h |
|---|---|---|
| AC | 0,74 | 12,76 |
| MAC | 1,10 | 12,79 |
| IPA | 0,062 | 1,05 |

| Produkte: | entstanden kg/h | Mol/h |
|---|---|---|
| OHM | 1,49 | 10,35 |
| AHDM | 0,17 | 0,73 |
| MO | 0,07 | 0,71 |

Selektivität OHM/AC = 81,1
Selektivität OHM/MAC = 80,9
Selektivität AHDM/MAC = 11,4
Selektivität MO/AC = 11,1

Beispiel 2:

Herstellung von 5-Oxohexannitril (OHN)

Im Unterschied zur Herstellung von OHM in Beispiel 1 wird das aus Kolonne (22) über Leitung (18) entnommene, hier hauptsächlich IPN enthaltende Gemisch, nicht ins Mischgefäss (1) zurückgeführt, sondern in einer Kolonne (25) in Isopropylamin und AN gespalten, welche ins Mischgefäss (1) eingespeist werden, wie Figur 3 zeigt. Ansonsten ist die Apparatur mit der in Beispiel 1 benutzten identisch.

In das Mischgefäss (1) werden pro Stunde über Leitung (2) 1,08 kg Acrylnitril, 10 g Benzoesäure und 4 g Hydrochinon, sowie über Leitung (3) 1,16 kg AC und über Leitung (4) 40 g Isopropylamin frisch zugeführt. Dazu kommen folgende Recyclierungsströme:

über Leitung (5) 6,3 kg AC, 0,4 kg AN, 45 g MO, 35 g IPN, 180 g $H_2O$;

über Leitung (17) 0,65 kg, bestehend hauptsächlich aus AC und AN.

Über Leitung (18a) werden Isopropylamin und AN in das Mischgefäss (1) eingespeist, die das Kopfprodukt von Kolonne (25) darstellen. In den Sumpf dieser Kolonne, die bei Normaldruck betrieben wird, werden 0,2 kg/h eines hauptsächlich aus IPN bestehenden Produktes eingespeist, das – wie eben erwähnt – über Leitung (18) aus Kolonne (22) entnommen wird. Zwischen 170 bis 200°C Sumpftemperatur spaltet IPN in Gegenwart von 1 bis 5% Benzoesäure[1)] nahezu quantitativ in Isopropylamin und AN. Von Zeit zu Zeit wird eine geringe Menge Hochsieder über Leitung (26) aus dem Sumpf von Kolonne (25) ausgeschleust.

Nach intensivem Mischen im Mischgefäss (1) wird das gebildete Ausgangsgemisch (10,06 kg/h, bestehend aus 80% AC, 15% ACN, 0,4% MO, 2,0% $H_2O$ und 2,5% IIPA) über Leitung (6) in das Strömungsrohr (7) gepumpt. Die Reaktortemperatur beträgt wieder 230°C, der Druck 55 bar. Das am Reaktorende stündlich anfallende Gemisch (68,0% AC, 4,2% ACN, 1,5% MO, 1,6% IPN, 18,6% OHN, 2,2% $H_2O$, 1,4% IIPA) wird über Leitung (19) direkt in den Verdampfer (20) entspannt, der bei Normaldruck und etwa 85°C Sumpftemperatur betrieben wird.

Dabei entsteht ein Destillat, das über Leitung (5) zum Mischgefäss recycliert wird und dessen Zusammensetzung bereits oben erwähnt wurde. Im Sumpf des Verdampfers (20) fallen stündlich 3,1 kg eines Gemisches der folgenden Zusammensetzung an: AC 20%; ACN 1%; MO 2%; IPN 5,5%; OHN 60%; AHDN 6%; $H_2O$ 1%.

Dieses Gemisch wird über Leitung (21) kontinuierlich in Kolonne (22) eingespeist. Der Druck am Kollonnenkopf beträgt 50 mbar; die Kopftemperatur beträgt −5°C, die Sumpftemperatur wird auf 140°C eingestellt. Das Rücklaufverhältnis beträgt $V_R = 3$. Am Kolonnenkopf, der mit Sole gekühlt wird, fallen stündlich etwa 0,65 kg eines Gemisches an, das hauptsächlich aus AC und ACN besteht und wie oben beschrieben über Leitung (17) zum Mischgefäss (1) zurückgeführt wird. Auf dem 2. Boden über dem Zulauf werden stündlich etwa 0,20 kg eines Gemisches abgenommen, das zu 75% aus IPN besteht, neben etwas OHN und MO. Dieses Gemisch wird wie beschrieben der Kolonne (25) zugeführt. Das Wasser-MO-Azeotrop (120 g) wird wieder 8 Böden oberhalb von Leitung (18) (IPN-Seitenabnahme) als zweiphasiges Gemisch über Leitung (23) ausgeschleust. Dadurch wird wieder der Wassergehalt des Ausgangsgemisches auf 1 bis 3 Gew.-% eingestellt.

Im Sumpf von Kolonne (22) fallen stündlich 2,25 kg eines Gemisches an, das, abgesehen von 0,2% IPN, völlig frei von Leichtsiedern ist. Neben 83% OHN sind darin 8% AHDN enthalten.

Dieses Gemisch wird über Leitung (24) kontinuierlich in die Kolonne (14) eingespeist, die bei 13 mbar Druck am Kolonnenkopf betrieben wird. Die Sumpftemperatur beträgt 170 bis 180°C. Am Kopf werden stündlich 1,86 kg 99%iges OHN bei einem Rücklaufverhältnis von $V_R = 0,1$ über Leitung (15) abgenommen.

Im Sumpf der Kolonne (14) fallen stündlich 0,39 kg eines Hochsiedergemisches an, das zu 50% aus AHDN besteht und über Leitung (16) entnommen wird.

Aus den stündlich dem System zugeführten Mengen und dem stündlich anfallenden OHN errechnen sich folgende Selektivitäten bezüglich AC bzw. ACN:

[1)] auch andere Säuren, beispielsweise p-Toluolsulfonsäure oder eine quaternäre Ammoniumbase wie Tetraethylammoniumhydroxid sind geeignet.

| Ausgangsmaterialien: | eingesetzt kg/h | Mol/h |
|---|---|---|
| AC | 1,16 | 20,0 |
| ACN | 1,08 | 20,4 |
| IPA | 0,040 | 0,68 |

Selektivität OHN/AC = 83,8
Selektivität OHN/ACN = 82,1
Selektivität AHDN/ACN = 10,4
Selektivität MO/AC = 10,0

| Produkte: | entstanden kg/h | Mol/h |
|---|---|---|
| OHM | 1,86 | 16,76 |
| AHDN | 0,18 | 1,1 |
| MO | 0,10 | 1,0 |

**Patentansprüche**

1. Verfahren zur Herstellung von 5-Oxohexansäurealkylester bzw. 5-Oxohexannitril durch Umsetzung von Aceton mit einem Acrylester, dessen Alkoholkomponente eine Kettenlänge von 1 bis 6 C-Atomen aufweist bzw. mit Acrylnitril bei erhöhter Temperatur in der Flüssigphase in Gegenwart eines primären Amins und/oder einer Schiffschen Base aus primärem Amin und Aceton und/oder einer Schiffschen Base aus primärem Amin und Aceton und/oder eines β-Aminopropionsäurederivats aus primärem Amin und Acrylester bzw. Acrylnitril sowie einer sauren Verbindung bei einem Druck oberhalb des Dampfdrucks des Reaktionsgemisches und unterhalb 500 bar, dadurch gekennzeichnet, dass das Reaktionsgemisch nach der Umsetzung kontinuierlich in eine Destillationskolonne eingespeist wird, über Kopf die Leichtsieder abdestilliert werden, aus der sich bildenden Anreicherungszone des Mesityloxid-Wasser-Azeotrops dieses abgezogen und aus dem zurückbleibenden Gemisch der 5-Oxohexansäurealkylester bzw. das 5-Oxohexannitril destillativ in derselben oder einer weiteren Kolonne gewonnen wird.

2. Verfahren nach Anspruch 1, dadurch gekennzeichnet, dass der in der Zone unterhalb der Anreicherungszone des Mesityloxid-Wasser-Azeotrops sich anreichernde Alkylester bzw. Nitril der β-Aminopropionsäure aus der Destillationskolonne abgezogen wird.

3. Verfahren zur Herstellung von 5-Oxohexansäuremethylester bzw. 5-Oxohexannitril durch Umsetzung von Aceton mit Methylacrylat bzw. Acrylnitril bei erhöhter Temperatur in der Flüssigphase in Gegenwart eines primären Amins und/oder einer Schiffschen Base aus primärem Amin und Aceton und/oder eines β-Aminopropionats aus primärem Amin und Methylacrylat bzw. Acrylnitril sowie einer sauren Verbindung, bei einem Druck oberhalb des Dampfdrucks des Reaktionsgemisches und unterhalb von 500 bar, dadurch gekennzeichnet, dass das Reaktionsgemisch nach der Umsetzung

a) von der Hauptmenge der bei Normaldruck unter 90°C siedenden Komponenten befreit wird, welche direkt in den Reaktionsraum zurückgeführt werden, und sodann
b) kontinuierlich in eine Destillationskolonne eingespeist wird, wo über Kopf der Rest der unter 90°C siedenden Komponenten abgenommen und in den Reaktionsraum zurückgeführt wird,
c) aus der Anreicherungszone des Mesityloxid-Wasser-Azeotrops dieses abgezogen und aus dem Prozess ausgeschleust wird,
d) aus der unterhalb der unter c) genannten Zone liegenden Anreicherungszone von β-Aminopropansäuremethylester bzw. β-Aminopropannitril diese Verbindung abgezogen und direkt oder nach Spaltung in Amin und Acrylverbindung in den Reaktor zurückgeführt wird,
e) aus dem verbleibenden Substanzgemisch der 5-Oxohexansäuremethylester bzw. das 5-Oxohexannitril in derselben oder einer nachgeschalteten Vakuumkolonne destillativ gewonnen wird.

4. Verfahren nach Anspruch 3, dadurch gekennzeichnet, dass die in den Schritten b), c) und d) eingesetzte Destillationskolonne unter Vakuum von 10 bis 500 mbar betrieben wird.

5. Verfahren nach Anspruch 1 bis 4, dadurch gekennzeichnet, dass das im Sumpf der Destillationskolonne anfallende Produkt kontinuierlich einer Vakuumkolonne zugeführt wird, wo über Kopf reiner 5-Oxohexansäurealkylester bzw. 5-Oxohexannitril anfällt und der Sumpf hauptsächlich Acetylheptandisäuredialkylester bzw. -dinitril sowie 5-Oxo-7-methyl-octen-6-säurealkylester bzw. 5-Oxo-7-methyl-octen-6-säurenitril enthält.

6. Verfahren zur Herstellung von 5-Oxohexansäuremethylester bzw. 5-Oxohexannitril durch Umsetzung von Aceton mit Methylacrylat bzw. Acrylnitril bei erhöhter Temperatur in der Flüssigphase in Gegenwart eines primären Amins und/oder einer Schiffschen Base aus primärem Amin und Aceton und/oder eines β-Aminopropionats aus primärem Amin und Methylacrylat bzw. Acrylnitril, sowie einer sauren Verbindung, bei einem Druck oberhalb des Dampfdrucks des Reaktionsgemisches und unterhalb von 500 bar, dadurch gekennzeichnet, dass das Reaktionsgemisch nach der Umsetzung

a) kontinuierlich in einem Verdampfer (20) zwischen 60 und 100°C vom Grossteil der Leichtsieder befreit wird, welche direkt recycliert werden,
b) sodann in eine bei 20 bis 130 mbar betriebenen Vakuumkolonne (22) eingespeist wird, wo über Kopf der Rest der Leichtsieder abgenommen und recycliert wird,
c) in einer Zone unmittelbar über dem Kolonnenzulauf Aminopropansäuremethylester bzw. Aminopropansäurenitril kontinuierlich abgenommen und direkt oder nach Zersetzung in Amin und Acrylverbindung recycliert wird,
d) oberhalb der Anreicherungszone vom Aminopropansäureester oder -nitril ein Azeotrop aus Mesityloxid und Wasser abgenommen und aus dem Prozess ausgeschleust wird,
e) das im Sumpf dieser Kolonne (22) anfallende Produkt kontinuierlich einer weiteren Vakuumkolonne (14) zugeführt wird, wo über Kopf reiner 5-Oxohexansäuremethylester bzw. 5-Oxohexannitril anfällt und im Sumpf hauptsächlich Acetylheptandisäuredimethylester bzw. -dinitril sowie 5-Oxo-7-methylocten-6-säure-methylester bzw. -nitril anfallen.

**Revendications**

1. Procédé de préparation d'oxo-5 hexanoates d'alkyles ou de l'oxo-5 hexane-nitrile par réaction de l'acétone avec un ester acrylique dont la composante alcool a une longueur de chaîne de 1 à 6 atomes de carbone ou avec l'acrylonitrile, à température élevée, en phase liquide, en présence d'une amine primaire et/ou d'une base de Schiff dérivant d'une amine primaire et de l'acétone et/ou d'un dérivé de l'acide β-amino-propionique provenant d'une amine primaire et d'un ester acrylique ou de l'acrylonitrile ainsi que d'un composé acide, sous une pression supérieure à la pression de vapeur du mélange réactionnel et inférieure à 500 bar, procédé caractérisé en ce qu'après la réaction on introduit continuellement le mélange réactionnel dans une colonne de distillation, on chasse par distillation, à la tête de la colonne, les constituants à bas point d'ébullition, on retire l'azéotrope oxyde de mésityle/eau de la zone d'enrichissement en celui-ci qui se forme et, du mélange restant, on isole l'oxo-5 hexanoate d'alkyle ou l'oxo-5 hexane-nitrile par distillation dans la même colonne ou dans une autre colonne.

2. Procédé selon la revendication 1, caractérisé en ce que l'ester alkylique ou le nitrile de l'acide β-amino-propionique qui se concentre dans la zone située au-dessous de la zone d'enrichissement en azéotrope oxyde de mésityle/eau est retiré dela colonne de distillation.

3. Procédé de préparation de l'oxo-5 hexanoate de méthyle ou de l'oxo-5 hexane-nitrile par réaction de l'acétone avec, respectivement, l'acrylate de méthyle ou l'acrylonitrile, à température élevée, en phase liquide, en présence d'une amine primaire et/ou d'une base de Schiff dérivant d'une amine primaire et de l'acétone, et/ou d'un dérivé de l'acide β-amino-propionique provenant d'une amine primaire et de l'acrylate de méthyle ou de l'acrylonitrile, ainsi que d'un composé acide, sous une pression supérieure à la pression de vapeur du mélange réactionnel et inférieure à 500 bar, procédé caractérisé en ce que:

a) après la réaction on élimine du mélange réactionnel la majeure partie des composants bouillant à une température inférieure à 90°C sous la pression normale, composantes que l'on renvoie directement dans l'enceinte réactionnelle, puis
b) on introduit le mélange réactionnel, en continu, dans une colonne de distillation, d'où l'on fait sortir, à la tête, le reste des composants bouillant au-dessous de 90°C, lesquelles sont renvoyées dans l'enceinte réactionnelle,
c) on retire l'azéotrope oxyde de mésityle/eau de la zone où cet azéotrope se concentre et on l'exclut du procédé,
d) on retire le β-amino-propanoate de méthyle ou le β-amino-propane-nitrile de la zone d'enrichissement en ce composé qui se trouve au-dessous de la zone dont il a été question sous c), et on renvoie ce composé au réacteur directement ou après l'avoir scindé en l'amine et le composé acrylique, et
e) du mélange restant on isole l'oxo-5 hexanoate de méthyle ou l'oxo-5 hexane-nitrile par distillation dans la même colonne sous vide ou dans une colonne sous vide montée en aval.

4. Procédé selon la revendication 3, caractérisé en ce que la colonne de distillation mise en jeu dans les étapes b), c) et d) fonctionne sous un vide de 10 à 500 mbar.

5. Procédé selon l'une quelconque des revendications 1 à 4, procédé selon lequel le produit obtenu dans le bas de la colonne de distillation est envoyé en continu à une colonne sous pression réduite, de la tête de laquelle on retire, à l'état pur, de l'oxo-5 hexanoate d'alkyle ou de l'oxo-5 hexane-nitrile et dont le produit du bas contient principalement de l'acétyl-heptanedioate de dialkyle ou de l'acétyl-heptane-dinitrile ainsi que de l'oxo-5 méthyl-7 octène-6 oate d'alkyle ou de l'oxo-5 méthyl-7 octène-6 nitrile.

6. Procédé de préparation de l'oxo-5 hexanoate de méthyle ou de l'oxo-5 hexane-nitrile par réaction de l'acétone avec l'acrylate de méthyle ou l'acrylonitrile, à température élevée, en phase liquide, en présence d'une amine primaire et/ou d'une base de Schiff dérivant d'une amine primaire et de l'acétone, et/ou d'un dérivé de l'acide β-amino-propionique provenant d'une amine primaire et de l'acrylate de méthyle ou de l'acrylonitrile, ainsi que d'un composé acide, sous une pression supérieure à la pression de vapeur du mélange réactionnel et inférieure 500 bar, procédé caractérisé en ce que:

a) après la réaction on élimine continuellement du mélange réactionnel, dans un évaporateur (20), à une température comprise entre 60 et 100°C, la majeure partie des constituants à bas point d'ébullition, que l'on recycle directement,
b) ensuite on introduit le mélange réactionnel dans une colonne sous vide (22) fonctionnant entre 20 et 130 mbar, d'où l'on retire, par sa tête, le reste des constituants à bas point d'ébullition, lesquels sont recyclés,
c) d'une zone située juste au-dessus de l'endroit d'alimentation de la colonne on retire continuellement l'amino-propanoate de méthyle ou l'amino-propane-nitrile, que l'on recycle directement

ou après décomposition en l'amine et le composé acrylique,
d) au-dessus de la zone d'enrichissement en ester amino-propanoïque ou en amino-propane-nitrile on retire un mélange azéotropique d'oxyde mésityle et d'eau, et on l'exclut du procédé,
e) on envoie le produit obtenu au bas de cette colonne (22), en continu, à une autre colonne sous pression réduite (14), de la tête de laquelle on retire l'oxo-5 hexanoate de méthyle pur ou l'oxo-5 hexane-nitrile pur et au bas de laquelle on obtient principalement l'acétyl-heptane-dioate de diméthyle ou l'acétyl-heptane-dinitrile ainsi que l'oxo-5 méthyl-7 octène-6 oate de méthyle ou l'oxo-5 méthyl-7 octène-6 nitrile.

**Claims**

1. A process for the preparation of a 5-oxohexanoic acid alkyl ester or 5-oxohexanonitrile by reacting acetone with an acrylic ester in which the alcohol component has a chain length of 1 to 6 C-atoms, or with acrylonitrile, at elevated temperature, in the liquid phase and in the presence of a primary amine and/or a Schiff's base formed from a primary amine and acetone, and/or a β-aminopropionic acid derivative formed from a primary amine and an acrylic ester or acrylonitrile, and an acid compound, under a pressure higher than the vapor pressure of the reaction mixture and less than 500 bars, characterized by feeding the reaction mixture continuously into a distillation column after the reaction, removing the low-boilers by distillation as a top product, stripping off the mesityl oxide/water azeotrope from the enrichment zone of the latter which is formed, and isolating the 5-oxohexanoic acid alkyl ester or the 5-oxohexanonitrile by distillation from the residual mixture in the same column or in a further column.

2. A process as claimed in claim 1, wherein the alkyl ester or nitrile of β-aminopropionic acid which becomes concentrated in the zone below the enrichment zone of the mesityl oxide/water azeotrope, is withdrawn from the distillation column.

3. A process for the preparation of 5-oxohexanoic acid methyl ester or 5-oxohexanonitrile by reacting acetone with methyl acrylate or acrylonitril, respectively, at elevated temperature in the liquid phase, in the presence of a primary amine and/or a Schiff's base formed from a primary amine and acetone and/or a β-aminopropionate formed from a primary amine and methyl acrylate or acrylonitrile, respectively, and an acid compound, under a pressure higher than the vapor pressure of the reaction mixture and less than 500 bars, characterized by

a) freeing the reaction mixture, after the reaction, from the bulk of the components boiling below 90°C under normal pressure, which are recycled to the reaction space direct, and then
b) feeding the reaction mixture continuously into a distillation column where the remainder of the components boiling below 90°C are taken off as a top product and recycled to the reaction space,
c) withdrawing the mesityl oxide/water azeotrope from the zone where it is concentrated and removing it from the process,
d) withdrawing the β-aminopropanoic acid methyl ester or β-aminopropanonitrile from the zone, below the zone mentioned under c), where these compounds are concentrated and recycling them to the reactor direct or after they have been split into amine and acrylic compound, and
e) isolating the 5-oxohexanoic acid methyl ester or 5-oxohexanonitrile from the mixture of substances remaining by distillation in the same vacuum column or in a subsequent vacuum column.

4. A process as claimed in claim 3, wherein the distillation column employed in stages b), c) and d) is operated under a vacuum of 10 to 500 mbars.

5. A process as claimed in any of claims 1 to 4, wherein the product obtained in the sump of the distillation column is fed continuously to a vacuum column where pure 5-oxohexanoic acid alkyl ester or 5-oxohexanonitrile is obtained as a top product and the sump contains mainly acetylheptanedioic acid dialkyl ester or dinitrile and 5-oxo-7-methyloct-6-enoic acid alkyl ester or 5-oxo-7-methyloct-6-enonitrile.

6. A process for the preparation of 5-oxohexanoic acid methyl ester or 5-oxohexanonitrile by reacting acetone with methyl acrylate or acrylonitrile, respectively, at elevated temperature in the liquid phase, in the presence of a primary amine and/or a Schiff's base formed from a primary amine and acetone and/or a β-aminopropionate formed from a primary amine and methyl acrylate or acrylonitrile, respectively, and an acid compound, under a pressure higher than the vapor pressure of the reaction mixture and less than 500 bars, characterized by
a) freeing the reaction mixture, after the reaction, from the bulk of the low-boilers continuously in a vaporizer (20) at between 60 and 100°C, the low-boilers being directly recycled,
b) then feeding the reaction mixture into a vacuum column (22) operated at a pressure of 20 to 130 mbars, where the remainder of the low-boilers are taken off as a top product and recycled,
c) continuously taking off aminopropanoic acid methyl ester or aminopropanonitrile in a zone immediately above the feed to the column and recycling this product direct or after it has been decomposed into amine and acrylic compound,
d) taking off an azeotrope composed of mesityl oxide and water above the zone in which aminopropanoic acid ester or nitrile are concentrated and removing this azeotrope from the process, and
e) feeding the product obtained in the sump of this column (22) continuously to a further vacuum column (14), where pure 5-oxohexanoic acid methyl ester or 5-oxohexanonitrile is obtained as a top product and the bottom product obtained is mainly acetylheptanedioic acid dimethyl ester or dinitrile and 5-oxo-7-methylocten-6-oic acid methyl ester or nitrile.

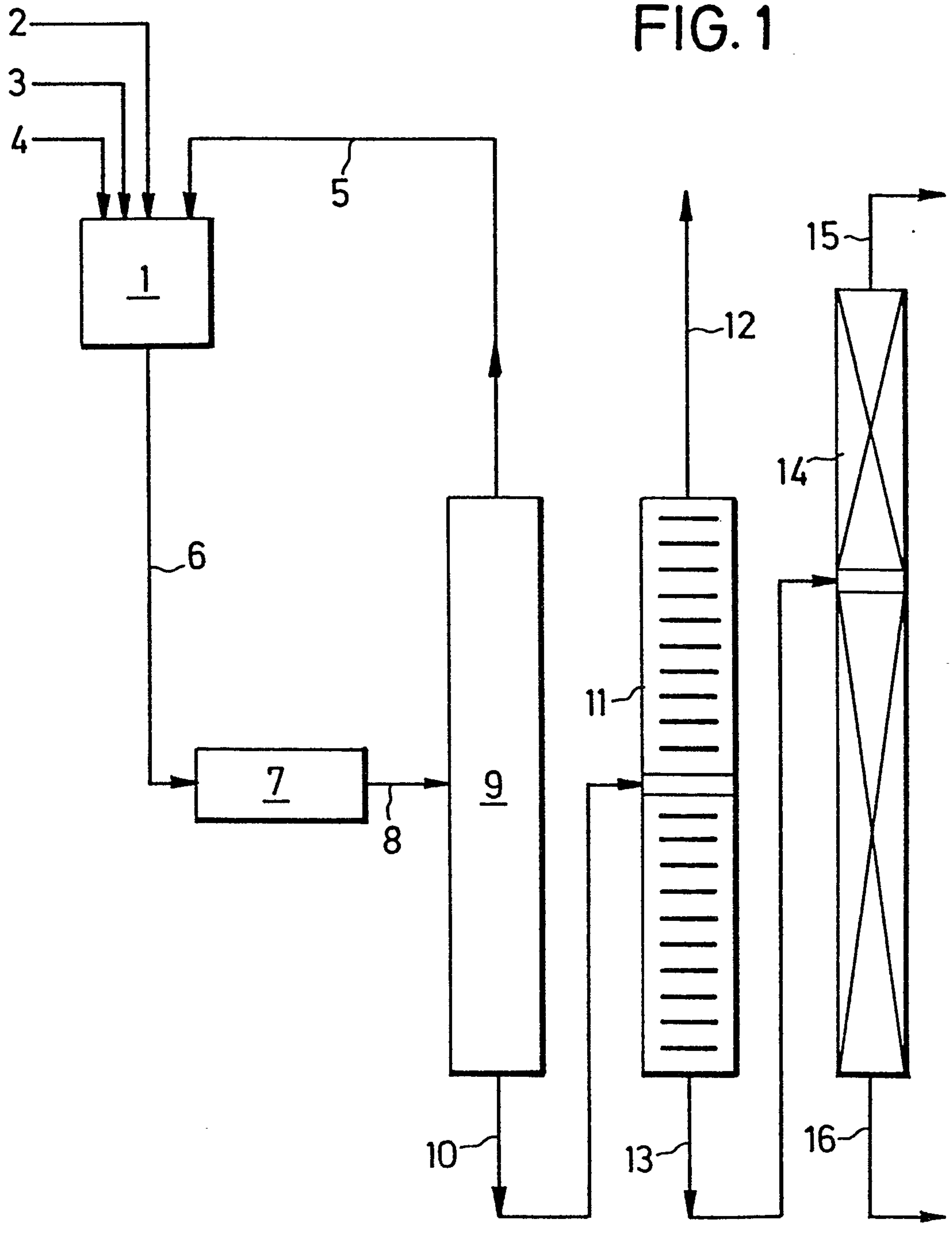


FIG. 1

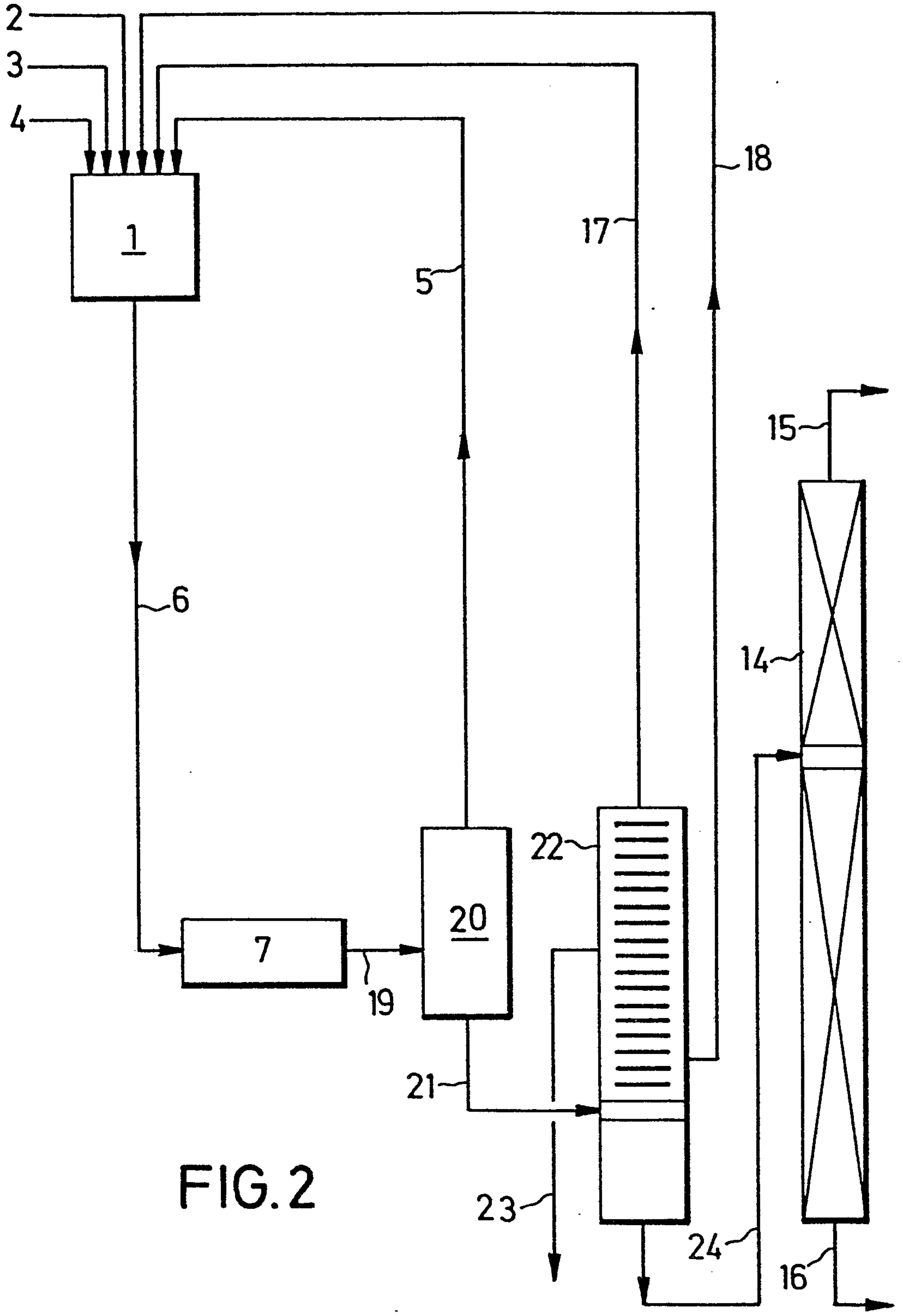


FIG. 2

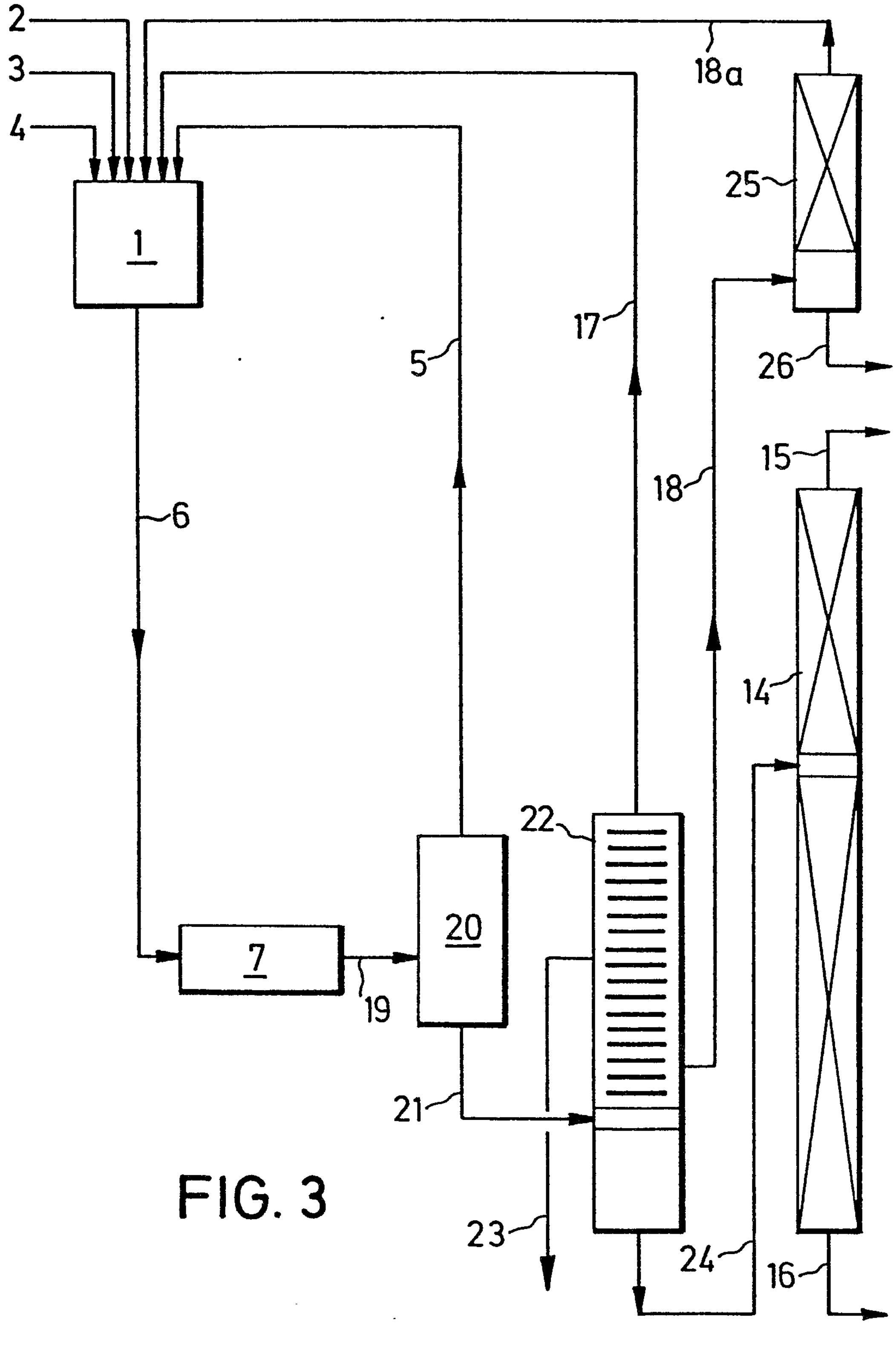


FIG. 3